Europäisches Patentamt

European Patent Office

Office européen des brevets

(11) Numéro de publication: **0 403 324**
**A1**

## (12) DEMANDE DE BREVET EUROPEEN

(21) Numéro de dépôt: **90401399.2**

(22) Date de dépôt: **25.05.90**

(51) Int. Cl.⁵: **A61B 5/113, A61M 16/00**

(30) Priorité: **29.05.89 FR 8907278**

(43) Date de publication de la demande:
**19.12.90 Bulletin 90/51**

(84) Etats contractants désignés:
**AT BE CH DE DK ES FR GB GR IT LI LU NL SE**

(71) Demandeur: **SOCIETE CIVILE D'ETUDES ET DE RECHERCHE REVO'AIR**
**Quartier du Pilon Blanc, Chemin de Miréio**
**F-13300 Salon de Provence(FR)**

(72) Inventeur: **Boutin, Guy**
**3 Place du Mont-Serein**
**F-30400 Villeneuve les Avignon(FR)**

(74) Mandataire: **Lhuillier, René et al**
**Cabinet Lepeudry, 6 rue du Faubourg St-Honoré**
**F-75008 Paris(FR)**

(54) **Dispositif de détection à micro-onde d'air, notamment pour le contrôle et la surveillance de la respiration.**

(57) Un tube (2) avec sa bille (5) utilisé en position horizontale ou légèrement inclinée est raccordé à un conduit (39) recueillant les variations de pression d'un respirateur nasal (53).

La détection du mouvement de va-et-vient de la bille au niveau du boîtier autonome de détection (10) renfermant le tube, est transmise par fibres optiques (45) à un module électronique de commande et d'alarme (22).

Application notamment à la surveillance respiratoire

fig.1

EP 0 403 324 A1

# Dispositif de détection à micro-onde d'air notamment pour le contrôle et la surveillance de la respiration.

L'invention se rapporte à un dispositif de détection à micro-onde d'air utilisable notamment pour contrôler, surveiller ou alarmer un sujet endormi, dès qu'apparaît un arrêt respiratoire anormal.

Dans le domaine de la surveillance respiratoire existent déjà un certain nombre de méthodes et de dispositifs qui malheureusement ne donnent pas toujours satisfaction pour les raisons exposées plus loin. Il s'avère que pour certaines personnes fragiles, ayant notamment des difficultés respiratoires ou des anomalies cardiaques, il soit nécessaire d'assurer le contrôle de leur sommeil et le déclenchement éventuel d'une alarme et/ou d'une distribution d'oxygène, et pour cela les dispositifs utilisés doivent être précis et fiables sans toutefois être contraignants.

Les systèmes utilisés jusqu'à présent reposent sur le principe de la mesure de la tension artérielle de l'individu, ou de la détection du mouvement de sa cage thoracique, ou encore de la mesure de la résistivité de la peau ou de la saturation en oxygène du sang. Dans les centres de réanimation, les hôpitaux et les cliniques, on dispose pour leur mise en oeuvre d'appareillages sophistiqués, lourds et volumineux, qui exigent donc la présence sur place du malade, et qui s'avèrent coûteux à l'achat, à l'entretien et pour le fonctionnement. On relèvera en outre que les appareils qui permettent le contrôle et la surveillance de la respiration par détection du mouvement de la cage thoracique ou de la résistivité de la peau, s'avèrent difficilement fiables du fait notamment que le paramètre de référence par rapport au déplacement à mesurer est mobile, que l'étalonnage est très difficile et, qu'en fin de compte ils manquent de sensibilité.

On leur préfère alors plutôt des appareillages portatifs, par exemple du type à mesurer la tension artérielle ou veineuse, ou à déterminer la saturation en oxygène du sang, mais ces appareillages, bien qu'ils fonctionnent correctement présentent l'énorme inconvénient de ne pouvoir agir sans réveiller le sujet et sans une intervention extérieure, alors que le manque d'oxygène a commencé à provoquer un malaise entraînant une accélération du rythme cardiaque et une montée de la tension.

Il existe enfin des systèmes basés sur la détection de la respiration elle-même, dans lesquels un détecteur est raccordé par un conduit à un masque ou à une sonde adapté au nez du patient, détecteur qui recueille les variations de pression de la respiration. On connaît ainsi, notamment par le US-A-3 817 238 un détecteur de respiration pour animal dans lequel l'organe de détection est constitué d'un tube transparent vertical raccordé par un conduit au museau de l'animal. Dans le tube, une bille légère se déplace au rythme de la respiration et son mouvement est détecté par une cellule photoélectrique soumise à l'éclairement d'une source lumineuse. Ce système fonctionne en tout ou rien et manque tout à fait de sensibilité, ne serait-ce que parce que la variation de pression d'air dans le tube doit être supérieure à la force de gravité qui s'exerce sur la bille dans ce tube vertical.

L'invention vise au contraire à obtenir un système qui soit extrêmement sensible à la moindre anomalie, ou à la moindre discontinuité dans le mouvement respiratoire normal, pour donner une alarme ou autoriser automatiquement une distribution d'oxygène, sans qu'une faible anomalie décelée entraîne systématiquement le réveil de la personne, et cela tout en étant d'une fiabilité et d'une simplicité de réalisation et de mise en oeuvre qui autorise son utilisation en toutes circonstances.

C'est pourquoi l'objet principal de la présente invention consiste en un dispositif de détection à micro-onde d'air utilisable notamment pour contrôler, surveiller ou alarmer un sujet endormi dès qu'apparaît un arrêt respiratoire anormal, et comportant un conduit recueillant les variations de la pression à contrôler, relié à au moins un tube renfermant une bille déplaçable, dispositif selon lequel le tube avec sa bille est utilisé en position horizontale ou légèrement inclinée, la bille se déplaçant avec un jeu minimum à l'intérieur du tube entre deux butées internes laissant un passage d'air, et la détection du mouvement de va-et-vient de la bille au niveau d'au moins un boîtier autonome de détection renfermant ledit tube étant transmise par des moyens de liaison à un module électronique de commande et d'alarme.

Selon une caractéristique particulière de l'invention, le boîtier de détection est traversé par une ouverture cylindrique servant de logement au tube interchangeable dont au moins une partie centrale est transparente, des moyens étant prévus pour le positionnement et le blocage dudit tube dans le boîtier. Un jeu de détecteurs est prévu de part et d'autre du tube, et en alignement avec le trajet de la bille pour détecter son va-et-vient.

Avantageusement, les moyens de liaison du boîtier de détection au module électronique de commande et d'alarme sont constitués de conduits à fibres optiques qui peuvent être réalisés en nappes intégrées dans ledit boîtier.

Selon une autre caractéristique particulière de l'invention, une électrovanne d'admission d'oxygène montée en sortie du détendeur d'une bouteille, délivre de l'oxygène, seulement durant la phase inspiratoire du sujet.

Le respirateur nasal utilisé comporte deux branches, l'une pour recueillir les variations de pression l'autre pour l'admission d'oxygène, chacune disposant d'un embout nasal et d'un embout buccal.

Selon une autre caractéristique de l'invention, on utilise deux tubes de verre alignés selon un axe légèrement incliné et montés en opposition qui sont reliés entre eux par un manchon souple et étanche à double sortie sur lequel est branché un conduit de dérivation qui recueille les variations de pression à contrôler.

En variante, ces variations de pression sont recueillies par un capteur de mouvement à membrane élastique et souple.

D'autres caractéristiques complémentaires, les avantages et les variantes de réalisation de l'invention ressortiront de la description qui va suivre d'exemples non limitatifs de réalisation dans lesquels on fait référence aux dessins annexés qui représentent :

Figure 1 une vue générale schématique du dispositif de détection

Figure 2 une vue en coupe du boîtier de détection

Figures 3 et 4, des vues de face et de profil d'un aérateur nasal

Figure 5, une vue schématique du montage de l'aérateur nasal

Figures 6 et 7, des vues de face et de profil du dispositif de détection

Figure 8, une variante de réalisation du boîtier de détection

Figure 9, une variante de capteur de mouvement

Figure 10, une vue schématique partielle, en perspective d'un bloc de détection analogique

et Figure 11, un schéma d'utilisation pour l'exploration en enceinte close.

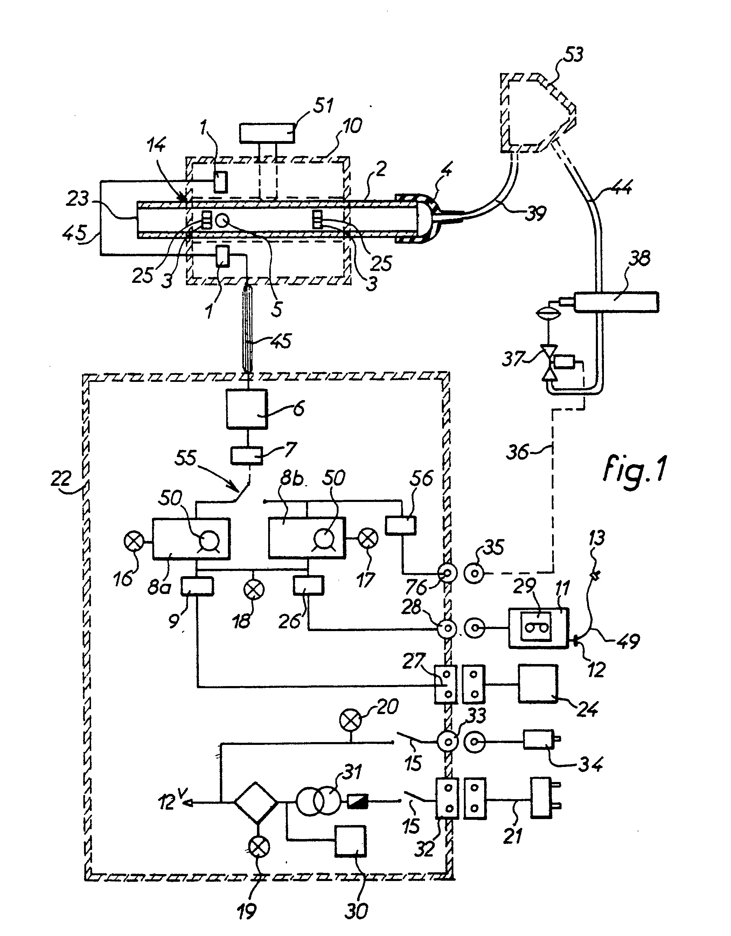

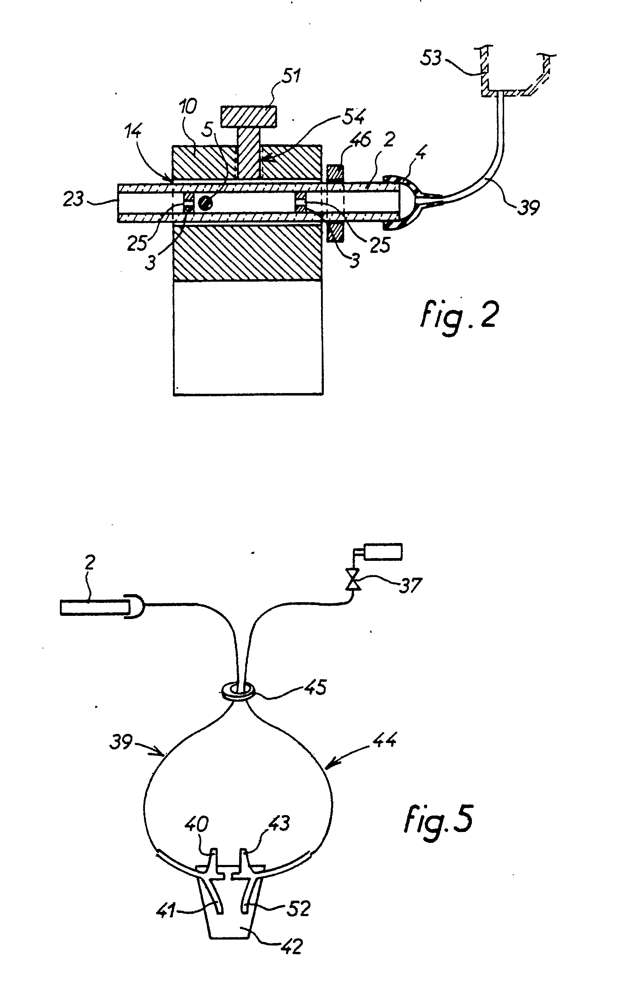

Le dispositif de détection à micro-onde d'air représenté schématiquement dans son ensemble à la figure 1 comporte plusieurs modules de base : - un boîtier autonome de détection 10 également représenté à la figure 2, qui est indépendant d'un module électronique de commande et d'alarme 22 auquel il est relié, et un respirateur nasal désigné dans son ensemble par la référence 53.

Dans la variante simplifiée représentée à la figure 2, on voit que le boîtier de détection 10 est traversé par une ouverture cylindrique horizontale 14 qui sert de logement et de support à un tube interchangeable en verre ou en matière plastique 2 dont au moins une partie centrale est transparente. Le tube 2 déborde de part et d'autre du boîtier 10. Une de ses extrémités 23 est ouverte à l'air libre. Par contre son autre extrémité ouverte est coiffée par un manchon souple et étanche 4 grâce auquel le tube est raccordé par un petit conduit 39 également interchangeable au respirateur nasal 53. Une rondelle annulaire 46 est emmanchée sur le tube 2, et joue le rôle de butée de positionnement en venant s'appliquer contre la paroi extérieure du boîtier de détection 10, pour assurer un bon centrage du tube dans le boîtier. Ce dernier est également traversé verticalement d'un orifice 54 qui communique avec l'ouverture 14 et qui permet d'y loger une vis de blocage 51 assurant l'immobilisation du tube 2 en position correcte dans le boîtier 10.

A l'intérieur du tube de verre 2 est placée une petite bille 5, très légère, par exemple en matière plastique ou fibre de carbone, dont le diamètre extérieur est très sensiblement égal au diamètre intérieur du tube pour que la bille puisse s'y déplacer avec un jeu minimum. La course de la bille est limitée dans le tube par deux petites butées internes 3 percées d'un orifice central 25, ou échancrées pour laisser passer l'air.

On voit à la figure 1 que le boîtier de détection 10 est équipé, de part et d'autre du tube 2 et à proximité d'une butée 3, d'un jeu de détecteurs 1 dont un des deux est par exemple une source lumineuse et l'autre une cellule photoélectrique, ou encore ce sont des détecteurs capacitifs ou résistifs. Ils sont de toute façon disposés de façon à détecter le déplacement de la bille 5 entre la position représentée qu'elle occupe en alignement avec les détecteurs et une autre position qu'elle pourrait prendre dans le tube. Ces détecteurs sont reliés par câbles électriques ou avantageusement par un conduit 45 à fibres optiques, pour transférer les signaux au module électronique de commande et d'alarme 22. A cet effet le conduit 45 aboutit à un amplificateur 6 dont le relais de sortie 7 selon la position du contact 55 se raccorde à l'un ou l'autre de deux temporisateurs 8 réglables par des boutons de commande 50. L'excitation des relais d'alarme 9 et 26 correspondant chacun à un temporisateur est provoquée par la tension d'alimentation gérée par une carte électronique comprenant les circuits temporisés réglables 8 qui reçoivent chacun une impulsion électrique provenant des détecteurs 1. Quand un contact du relais 7 est bloqué en position fermée ou ouverte, selon la fonction choisie, la temporisation commence son comptage pour se metre en alarme à la fin du temps préréglé par l'utilisateur à l'aide du bouton 50.

Le mouvement de la bille 5 dans le tube 2 est provoqué à partir du respirateur nasal 53 qui transmet, par le conduit 39 de faible diamètre, l'onde d'air résultant de l'inspiration ou de l'expiration du sujet à surveiller, au tube en question. Les détecteurs 1 détectent donc le mouvement de va et vient de la bille 5 se déplaçant avec un jeu minimum dans le tube 2 entre les deux butées d'arrêt 3.

Ainsi en cas d'arrêt respiratoire, c'est-à-dire en cas d'arrêt ou de modification du mouvement de va-et-vient de la bille, l'alarme est déclenchée. Si le sujet se remet à respirer, la bille reprend son mouvement de va-et-vient dans le tube et l'alarme s'arrête. Puis le cycle recommence.

Des moyens de signalisation et d'alarme restituent les informations recueillies par le module 22.

La tension d'utilisation donnée par le contact du relais d'alarme 26 peut être utilisée pour alimenter un émetteur 11 de bip sonore ou encore un écouteur d'oreille 13 raccordé à l'émetteur 11 par l'intermédiaire d'un fil d'écouteur 49 à un jack 12 effaçant le bip sonore. En variante elle peut servir aussi à assurer un enregistrement de bip sur cassette, pour une alarme individuelle, ou alimentée par un magnétophone à cassettes 29. Un report d'alarme peut également être exploité à distance et raccordé à la tension d'utilisation du deuxième relais 9 par l'intermédiaire d'un moyen d'interliaison 24 relié à la fiche 27 de sortie du module d'alarme.

En outre on trouve sur la face avant du module d'alarme 22, un interrupteur marche-arrêt 15, un socle 32 de prise de courant 220 volts, un voyant lumineux 16 en liaison avec un temporisateur réglable 8a pour signaler le premier top de temporisation, ainsi qu'un autre voyant lumineux 17 en liaison avec l'autre temporisateur réglable 8b pour signaler le second top de temporisation. De plus un voyant d'alarme 18 est asservi aux relais d'alarme (9,26) et un voyant témoin 20 permet de visualiser la marche d'une batterie rechargeable 30 reliée à un socle 33 de prise de courant 12 volts raccordé par exemple à un allume-cigare 34 de véhicule. Pour cela est prévu un ensemble 31 d'alimentation stabilisé et filtré 220 V/12 V avec fusibles, chargeur 12 volts incorporé, et voyant de contrôle 19.

Avantageusement, le détecteur est miniaturisé pour le transport en voyage, l'appareillage de détection fonctionnant alors sur pile autonome et pouvant se loger dans un mini-boîtier contenant également le tube de détection amovible.

La surveillance à distance du sujet peut être effectuée par retransmission de l'alarme par le réseau 220 volts du domicile, de l'hôpital ou de la clinique, sur le principe de la transmission HF 220 volts, employé pour les interphones. On utilise alors le moyen d'interliaison 24, comme émetteur relié à la fiche 27, recevant l'alimentation 220 volts, lorsque l'alarme se déclenche. Dans ce cas, un récepteur accordé sur la fréquence d'émission de l'alarme du détecteur d'arrêt respiratoire pourra être branché sur n'importe quelle prise de courant reliée au réseau de distribution électrique 220 volts sur lequel est raccordé le détecteur à la sortie 27

piloté par le relais 9.

L'émetteur de bip 11 ou le magnétophone 29 ainsi que l'émetteur du genre interphone 220 volts constitué du moyen d'interliaison 24, peuvent être incorporés au module.

Quand une insuffisance respiratoire nécessite un apport d'oxygène, une économie substantielle de ce produit peut être obtenue grâce au dispositif de l'invention par le fait que cet apport n'est fait que seulement durant la phase inspiratoire du sujet. On prévoit à cet effet une fiche 76 de sortie 12 volts sur le module de commande 22, qui permet par un raccord 35 et un câble 36 d'alimenter une électrovanne 37 d'admission d'oxygène montée en sortie du détendeur d'une bouteille 38. La fiche 76 est alimentée par un relais 56 relié au temporisateur réglable 8b, qui est excité seulement par la détection de la phase inspiratoire.

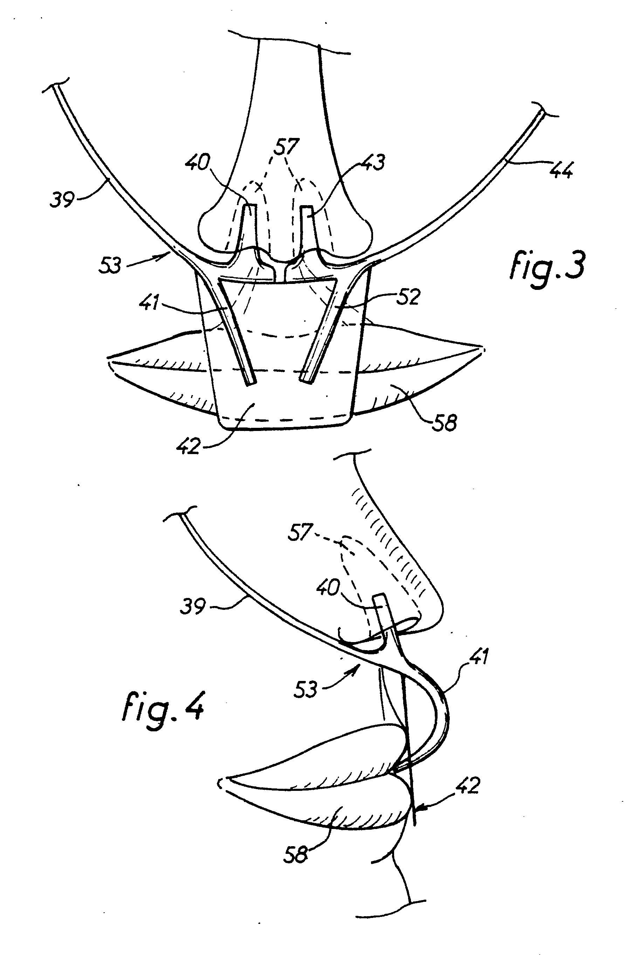

Le respirateur nasal 53 est représenté plus en détail aux figures 3, 4 et 5. Il comporte deux branches symétriques 39 et 44.

La branche 39 raccordée, comme indiqué précédemment, au tube 2 par l'intermédiaire du manchon 4 se divise vers, d'une part un embout nasal 40 destiné à être introduit dans une narine 57 du sujet, et d'autre part, en dérivation un embout buccal 41, destiné à être introduit dans la bouche 58 du sujet. L'extrémité de la branche 39 avec ses embouts 40,41 est fixée sur un support plat et souple 42 qui est percé pour permettre à l'embout buccal 41 de recevoir l'inspiration ou l'expiration d'un sujet dormant occasionnellement la bouche ouverte. Le support 42 se trouve à cheval sur les lèvres fermées du sujet.

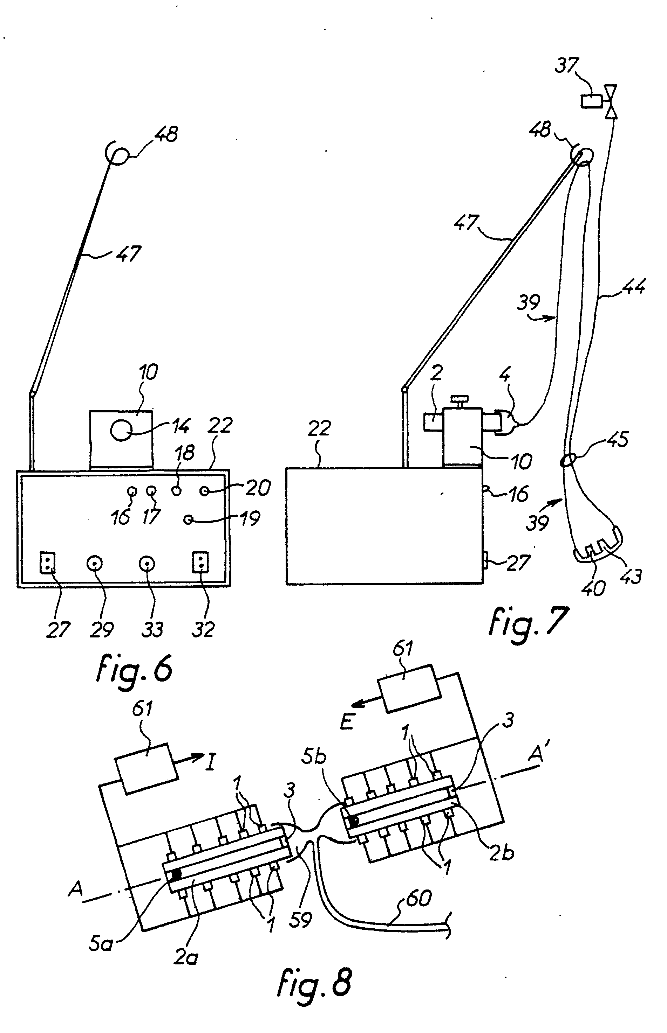

L'autre branche 44 du respirateur nasal 53 est raccordée à la sortie de l'électrovanne 37 (figure 1) permettant la distribution d'oxygène. Cette branche se termine également par un embout nasal 43 pénétrant dans l'autre narine, ainsi que par un autre embout buccal 52 en dérivation. La branche 44 et ses embouts 43 et 52 est aussi fixée de la même façon sur le support plat et souple 42. Le positionnement des embouts nasaux et buccaux est donc correctement assuré. Une bague plastique coulissante 45 permet de serrer les deux branches 39 et 44 derrière la tête, pour le maintien du respirateur nasal, comme on le voit plus précisément à la figure 5.

On voit à la figure 3 que les deux branches 39 et 44 du respirateur nasal 53 sont indépendantes l'une de l'autre, puisque raccordées à des circuits différents.

En variante de réalisation, les deux embouts nasaux 40 et 43 pourraient être raccordés aux deux branches 39 et 44 mises alors en communication pour l'utilisation de l'embout nasal complet, soit en détection respiratoire, soit en insufflation d'oxygène sans économiseur comme le montre plus précisément la figure 7.

Selon encore une variante non représentée et pour permettre à la fois la détection et l'oxygénothérapie, le masque pourrait être constitué de doubles embouts nasaux et buccaux, correspondant à des circuits séparés.

Les figures 6 et 7 illustrent un mode particulier de réalisation du dispositif de détection à micro onde d'air décrit précédemment. On reconnaît le module électronique de commande et d'alarme 22 qui se présente comme un boîtier portant sur une de ses faces les différentes fiches ou voyants évoqués plus haut. Au-dessus de ce module est posé le boîtier de détection 10 équipé de son tube 2.

Un support télescopique articulé 47 est fixé de façon escamotable sur le module 22 dont l'extrémité supérieure se termine par un anneau 48 dit en "queue de cochon" dans lequel sont engagés les tubes souples 39 et 44 de l'aérateur nasal ainsi qu'éventuellement d'autres fils tels que le fil d'écouteur 49 pour l'écoute à l'oreille ou au casque. Ces tubes ou fils sont donc maintenus en hauteur par cet anneau et ne peuvent gêner le sujet endormi. On notera que le boîtier 10 est autonome et pourrait être placé ailleurs, voire à une certaine distance du module, puisque de toute façon il n'est raccordé au module que par un conduit 45 avantageusement à fibres optiques.

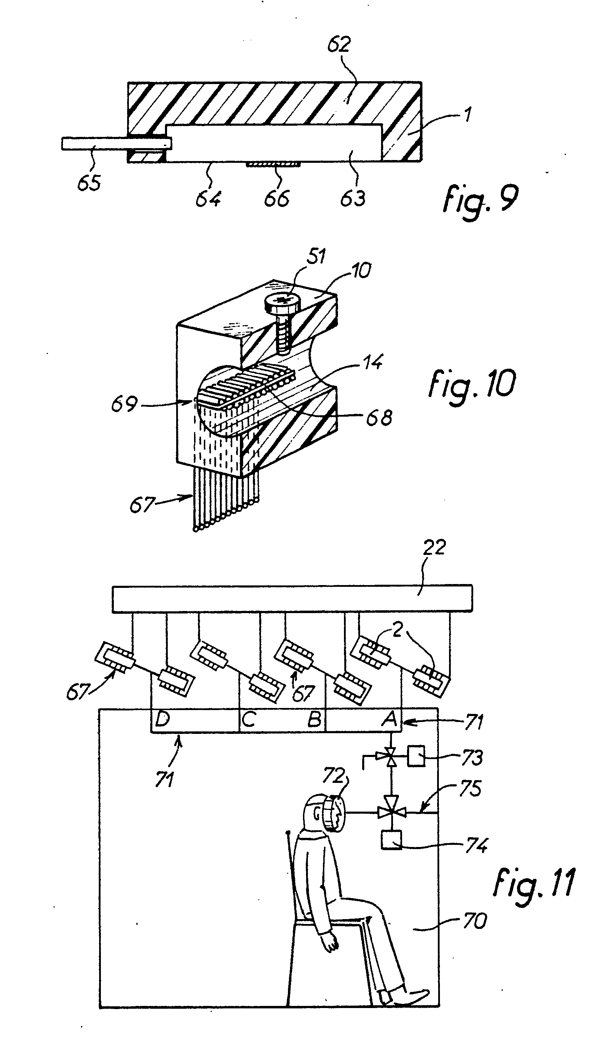

Quand il s'agit donc de transmettre les informations par fibres optiques, on utilise avantageusement un bloc de détection analogique tel que représenté à la figure 10.

Le bloc 10 est aussi traversé par l'ouverture 14 servant à loger le tube de verre non représenté, également maintenu par sa vis de blocage 51. Une nappe 67 de fibres optiques 45 est noyée dans la masse du bloc et débouche par une lumière longitudinale 68 dans la cavité que constitue l'ouverture 14, après une partie 69 coudée à 90°. Une autre nappe de fibres optiques, symétrique de la première, et non représentée pour la clarté de la figure, débouche en face par une autre lumière longitudinale. Ainsi d'un côté se trouve une série de fibres émettrices de lumière et de l'autre une série de fibres réceptrices raccordées au module 22.

En variante de réalisation les fibres optiques pourraient être remplacées par un dispositif de reconnaissance de forme par exemple composé de barrettes de cellules sensibles associées à un dispositif d'intelligence artificielle.

En outre la prise de détection de la respiration constituée du manchon étanche 4 coiffant le tube 2, qui est relié par le conduit 39 à l'aérateur nasal 53, peut s'effectuer à longue distance du sujet. En effet le conduit 39 est de faible section et le volume d'air contenu est peu important. Un conduit 39 de grande longueur ne perturberait pas les micro-ondes d'air générées par le sujet. C'est un avantage intéressant qui autorise l'usage du dispositif dans des conditions particulières, par exemple pour des personnes travaillant ou évoluant en atmosphère dangereuse ou explosive, car il permet un contrôle à distance de la régularité de leur respiration permettant d'effectuer un secours en cas d'anomalie respiratoire.

On a décrit l'invention sur la base d'une version simplifiée de réalisation d'un boîtier de détection tel qu'illustré à la figure 2 dans laquelle le tube 2 est présenté en position horizontale. L'invention ne se limite pas à cette variante particulière mais englobe également toute réalisation du boîtier dans lequel le tube serait en position faiblement inclinée, le mot "faiblement" définissant une pente qui soit juste suffisante et nécessaire pour que la force de gravité puisse s'exercer sur la bille légère 5, à l'encontre des forces d'inertie et de frottement dues à son faible jeu, afin que ladite bille, quand elle n'est plus soumise à l'inspiration ou à l'expiration du sujet, revienne par son propre poids vers la partie basse du tube incliné. Avec une faible inclinaison du tube de verre, on évite que la bille 5 ne colle sur l'une ou l'autre des butées 3 du fait qu'elle se trouve appliquée sur une butée lors de chaque inspiration et expiration. En outre on augmente ainsi la sensibilité de l'appareil, ce qui permet de l'étalonner.

Pour mieux comprendre le fonctionnement du dispositif à tube incliné, on décrit ci-après un mode particulier de réalisation faisant référence à la figure 8.

Deux tubes de verre 2a et 2b alignés selon un axe AA' légèrement incliné, de façon réglable, sur l'horizontale, sont reliés entre eux par un manchon souple et étanche 59 à double sortie, sur lequel un conduit de dérivation 60 relie les deux tubes au respirateur nasal. Chaque tube est également équipé de butées 3. Plusieurs jeux de détecteurs 1 du type optoélectroniques ou à nappes de fibres optiques sont disposés tout du long de chaque tube, chaque détecteur étant relié à un boîtier électronique 61 destiné à mémoriser la position de la bille 5 dans chaque tube.

Dans ce dispositif à deux tubes inclinés montés en opposition, la gravité maintient la bille en position basse comme représenté, c'est-à-dire que la bille 5a est du côté de l'extrémité ouverte du tube 2a tandis que la bille 5b est à côté du manchon 59 dans le tube 2b. A l'inspiration du sujet par le tube 60, la bille 5b restera immobile, mais la bille 5a remontera dans le tube de verre 2a sur une longueur proportionnelle à l'intensité de l'inspiration, pour venir au maximum contre la butée haute, longueur qui sera détectée par le détecteur correspondant. Au contraire, à l'expiration la bille 5a reviendra à sa position première sous l'effet de l'onde d'air mais aussi de la gravité, tandis que la bille 5b sera repoussée dans le tube de verre 2b sur une longueur proportionnelle à l'intensité de l'expiration, longueur également détectée par le détecteur correspondant.On obtient ainsi en sortie du boîtier électronique 61 correspondant au tube 2a réagissant à l'inspiration I du sujet, et en sortie de l'autre boîtier 61 correspondant au tube 2b réagissant à l'expiration E du sujet, un signal analogique ou numérique utilisable sur l'entrée interface d'un ordinateur ou tout autre appareil de mesure approprié, pour l'enregistrement, le contrôle et la mesure du débit de la respiration aussi bien à l'inspiration qu'à l'expiration. On peut alors obtenir une courbe ou un graphique pour déterminer le volume thoracique, ainsi que les actions ventilatoires des organes de la respiration, notamment pour la mesure des faibles débits d'air respiratoires dans le cas de la détection des hypopnées.

Ce dispositif est extrêmement sensible à la moindre micro onde d'air établie dans le conduit 60, d'autant que la série des capteurs 1 permet de contrôler le moindre déplacement de la bille 5 qui, on le sait, est très légère et se déplace avec un faible jeu. Le conduit en question peut donc être relié à un autre système de détection que le respirateur nasal précédemment décrit. Ainsi on pourrait en utilisant le montage de la figure 8 déceler le mouvement du coeur par la détection du mouvement du thorax ou de l'abdomen, ou le battement d'une artère, à condition de disposer d'un capteur très sensible, générant une micro onde d'air qui puisse se substituer au respirateur nasal.

L'invention prévoit à cet effet, en variante, un capteur de mouvement tel que représenté à la figure 9.

Le capteur en question est constitué d'un corps rigide annulaire 62 formant un boîtier plat dont la chambre interne 63 est fermée par une membrane mince élastique et souple 64 avantageusement réalisée en caoutchouc ou matériau analogue. Le corps 62 est percé d'un orifice équipé d'un tube de raccordement 65 sur lequel sera emmanché le conduit souple 60 précédemment évoqué menant aux tubes inclinés, de façon à faire communiquer la chambre 63 avec lesdits tubes. Sur la membrane 64 est éventuellement rapporté un petit aimant permanent 66 dont la fonction apparaîtra plus loin.

La membrane étant bien tendue sur le capteur, à la façon d'un stéthoscope, le moindre battement ou mouvement de la partie du corps sur laquelle il est appliqué crée une pression ou une dépression dans la chambre 63, en déformant la membrane élastique 64, ce qui, grâce à la liaison 60 provoque le mouvement de va-et-vient des billes dans leur tube de verre.

Ce capteur peut être utilisé comme interrupteur pneumo électronique, ou encore comme interrupteur magnétique pneumo électronique du fait de l'aimant permanent 66, autorisant la détection d'objets métalliques sans contact direct du capteur avec lesdits objets.

Un tel dispositif de détection peut aussi être utilisé pour une exploration fonctionnelle de la respiration en enceinte close, comme l'illustre la figure 11.

Dans une enceinte close 70 parfaitement étanche, à l'intérieur de laquelle se trouve le sujet à surveiller, aboutissent des tubes de liaison 71 provenant de quatre jeux A,B,C,D de double tubes de mesures 2 à inclinaison réglable. Les tubes 71 aboutissent à un masque respiratoire étanche 72 porté par le sujet, par l'intermédiaire d'une électrovanne trois voies 73 pour la sélection de la mesure de l'expansion thoracique, ainsi que d'une autre électrovanne trois voies 74 pour la sélection de la mesure du volume inspiratoire et expiratoire réel par un tube 75 de communication directe avec l'extérieur.

Chaque tube 2 est équipé d'une nappe 67 de fibres optiques aboutissant à un module 22 assurant l'amplification, et autorisant l'intégration des signaux analogiques et de mesure. Chaque jeu A,B,C,D de tubes inclinés est étalonné, par exemple en faisant varier l'inclinaison de l'un ou l'autre tube, pour correspondre à un niveau déterminé des débits mesurés. On peut ainsi mesurer le volume inspiratoire et expiratoire réel par communication directe de la respiration du sujet qui passe complètement par les quatre échelles de mesure.

Enfin en combinant un ou des capteurs thoraciques ou abdominaux mentionnés plus haut, avec un capteur nasal ou buccal, on pourrait aisément mesurer et contrôler à la fois le rythme et le volume respiratoire d'un sujet.

On voit que le ou les dispositifs de détection à micro onde d'air précédemment décrits trouvent des applications particulièrement séduisantes dans le domaine du contrôle et de la surveillance de la respiration, mais ils ne se limitent pas à ces utilisations spécifiques, toutes variantes d'utilisation mettant en oeuvre les moyens de détection d'un mouvement par micro onde d'air à l'aide d'un tube horizontal ou faiblement incliné, faisant également partie de l'invention

## Revendications

1.- Dispositif de détection à micro onde d'air utilisable notamment pour contrôler, surveiller ou alarmer un sujet endormi dès qu'apparaît un arrêt respiratoire anormal, et comportant un conduit recueillant les variations de la pression à contrôler, relié à au moins un tube renfermant une bille déplaçable, caractérisé en ce que le tube (2) avec sa bille (5) est utilisé en position horizontale ou légèrement inclinée, en ce que la bille se déplace avec un jeu minimum à l'intérieur du tube entre deux butées internes (3) laissant un passage d'air, et en ce que la détection du mouvement de va-et-vient de la bille au niveau d'au moins un boîtier autonome de détection (10) renfermant ledit tube est transmise par des moyens de liaison (45) à un module électronique de commande et d'alarme (22).

2.- Dispositif de détection selon la revendication 1, caractérisé en ce que le boîtier de détection (10) est traversé par une ouverture cylindrique (14) servant de logement au tube interchangeable (2) dont au moins une partie centrale est transparente, des moyens (46,51) étant prévus pour le positionnement et le blocage dudit tube dans le boîtier.

3.- Dispositif de détection selon la revendication 1, caractérisé en ce que le passage d'air se fait au travers des butées internes (3) par des orifices centraux (25)

4.- Dispositif de détection selon les revendications 1 et 2, caractérisé en ce qu'au moins un jeu de détecteurs (1) est prévu de part et d'autre du tube (2) et en alignement avec le trajet de la bille (5), pour détecter son va-et-vient.

5.- Dispositif de détection selon la revendication 1, caractérisé en ce que les moyens de liaison du boîtier de détection (10) au module électronique de commande et d'alarme (22) sont constitués de conduits à fibres optiques (45).

6.- Dispositif de détection selon les revendications 1, 2 et 5, caractérisé en ce que le boîtier 10 est constitué d'un bloc de détection analogique dans lequel sont noyées deux nappes (67) de fibres optiques (45) qui débouchent l'une en face de l'autre par des lumières longitudinales (68) dans la cavité que constitue l'ouverture (14).

7.- Dispositif de détection selon la revendication 1, caractérisé en ce que les moyens de liaison du boîtier de détection (10) au module électronique de commande et d'alarme (22) sont constitués d'un dispositif de reconnaissance de forme à cellules sensibles associées à un dispositif d'intelligence artificielle.

8.- Dispositif de détection selon les revendications 1 et 4, caractérisé en ce que le module électronique de commande et d'alarme (22) renferme au moins deux temporisateurs réglables (8) correspondant à des relais d'alarme (9) et (26) dont l'excitation est provoquée par une tension d'alimentation gérée par une carte électronique, à la suite d'émission d'impulsions générées par les détecteurs (1), et en ce que des moyens de signalisation (16,17,18,19,20) et/ou d'alarme (11,13,24,29) restituent les informations recueillies par ledit module de commande et d'alarme.

9.- Dispositif de détection selon la revendication 8, caractérisé en ce que le module de commande et d'alarme (22) porte au moins des voyants lumineux (16,17) liés aux tops de temporisation, des voyants d'alarme et/ou de contrôle (18,19) et un voyant (20) témoin de charge de batterie.

10.- Dispositif de détection selon la revendication 8, caractérisé en ce que le module de commande et d'alarme (22) porte au moins un émetteur de bip sonore d'alarme (11,13), un enregistreur à cassettes (29) et un moyen d'interliaison (24) pour report d'alarme.

11.- Dispositif de détection selon les revendications 1 et 8, caractérisé en ce que la transmission de l'alarme à distance s'effectue par le moyen d'interliaison (24) servant d'émetteur et recevant une alimentation 220 volts après déclenchement de l'alarme et réception de celle-ci sur un récepteur accordé sur la fréquence d'émission de l'émetteur.

12.- Dispositif de détection selon la revendication 1, caractérisé en ce qu'une électrovanne (37) d'admission d'oxygène montée en sortie du détendeur d'une bouteille (38), délivre de l'oxygène seulement durant la phase inspiratoire du sujet.

13.- Dispositif de détection selon les revendications 1 et 12, caractérisé en ce que le conduit (39) lié au tube (2) recueille les variations de pression d'un respirateur nasal (53) à deux branches dont l'autre branche (44) est raccordée à la sortie de l'électrovanne (37) d'admission d'oxygène.

14.- Dispositif de détection selon la revendication 13, caractérisé en ce que les branches (39) et (44) du respirateur nasal (53) se divisent chacune vers respectivement un embout nasal (40,43) et un embout buccal (41,52).

15.- Dispositif de détection selon les revendications 13 et 14, caractérisé en ce qu'un support (42), se trouvant à cheval sur les lèvres fermées du sujet, est prévu pour maintenir les branches (39,44) et leurs embouts.

16.- Dispositif de détection selon les revendications 13 et 14, caractérisé en ce que les embouts nasaux (40,43) sont raccordés aux deux branches (39,44) mises en communication pour l'utilisation de l'embout nasal complet soit en détection respiratoire, soit en insufflation d'oxygène.

17.- Dispositif de détection selon la revendication 1, caractérisé en ce que le conduit (39) recueillant les variations de la pression à contrôler est de faible section et de grande longueur pour permettre la détection d'un signal à distance sans perturber la micro onde d'air.

18.- Dispositif de détection selon la revendication 1, caractérisé en ce qu'on utilise deux tubes de verre (2a, 2b) alignés selon un axe AA' légèrement incliné et montés en opposition qui sont reliés entre eux par un manchon souple et étanche (59) à double sortie sur lequel est branché un conduit de dérivation (60) qui recueille les variations de pression à contrôler.

19.- Dispositif de détection selon les revendications 1 et 18, caractérisé en ce que le conduit (39,60) recueille les variations de pression d'un capteur de mouvement (62) dont une membrane élastique et souple (64) ferme une chambre interne (63), membrane dont le déplacement provoque une micro-onde d'air recueillie par les tubes (2).

20.- Dispositif de détection selon la revendication 19, caractérisé en ce qu'un aimant permanent (66) est associé à la membrane (64) pour l'utilisation du capteur comme détecteur à distance d'objets métalliques.

21.- Dispositif de détection selon les revendications 1 et 18, caractérisé en ce que plusieurs jeux de double tubes de mesure (2) sont reliés à un sujet par l'intermédiaire d'électrovannes (73,74) de mesure d'expansion thoracique et/ou de volume inspiratoire ou expiratoire,pour l'exploration fonctionnelle de la respiration en enceinte close.

22.- Dispositif de détection selon les revendications 1 et 18, caractérisé en ce que le système de détection est jumelé à un système de détection du rythme cardiaque et/ou de mesure d'oxygénation du sang.

*Fig.1*

fig. 2

fig. 5

fig.3

fig.4

EP 0 403 324 A1

fig.6

fig.7

fig.8

fig. 9

fig. 10

fig. 11

Office européen
des brevets

**RAPPORT DE RECHERCHE EUROPEENNE**

Numero de la demande

EP   90 40 1399

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-4 417 589  (W.E. FAVALORO) <br> * figure 3; colonne 2, lignes 1-42 * | 1 | A 61 B   5/113 <br> A 61 M  16/00 |
| Y | US-A-3 817 238  (L.R. MATSON) <br> * figure 2; colonne 1, lignes 40-62 * | 1 | |
| Y | FR-A-1 025 433  (A.M. WALTER) <br> * figure 1; page 1, colonne 2, lignes 10-31 * | 1 | |
| Y | US-A-3 347 222  (C.W. KOHRER) <br> * figure 1; abrégé * | 1 | |
| A | US-A-4 657 025  (C. ORLANDO) <br> * figure 6; colonne 9, lignes 21-34 * | 11,22 | |
| A | EP-A-0 285 470  (L'AIR LIQUIDE) <br> * figure 1; abrégé * | 12 | |
| A | US-A-4 366 821  (E.A. WITTMAIER et al.) <br> * figure 1; colonne 3, lignes 12-14 * | 11 | DOMAINES TECHNIQUES RECHERCHES (Int. Cl.5) |
| A | US-A-4 602 644  (J.P. DI BENEDETTO et al.) <br> * figure 4; abrégé * | 13 | A 61 B <br> A 61 M |
| A | GB-A-2 005 034  (A. SALVIDGE) <br> * figure 1; page 2, lignes 11-43 * | 1,20 | |

Le présent rapport a été établi pour toutes les revendications

| Lieu de la recherche | Date d'achèvement de la recherche | Examinateur |
|---|---|---|
| BERLIN | 05-09-1990 | BREUSING J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)